# EUROPÄISCHE PATENTANMELDUNG

(11) **EP 3 202 380 A1**
(43) Veröffentlichungstag der Anmeldung: **09.08.2017**
(21) Anmeldenummer: 17154577.5
(22) Anmeldetag: 03.02.2017
(51) Int. Cl.: A61F 13/00, A61B 17/132, A61F 13/02

(54) **WUNDABDECKUNG**

(30) Priorität: 06.02.2016 DE 102016102106
(71) Anmelder: Pedersen, Finn, 48291 Telgte (DE)
(72) Erfinder: Pedersen, Finn, 48291 Telgte (DE)
(74) Vertreter: Bungartz Christophersen Partnerschaft mbB Patentanwälte

(57) **Zusammenfassung**

Um bei einer Wundabdeckung eine etwaige Blutung durch einen gesteigerten Druck eines Abdeckpolsters auf den Wundbereich besser stillen zu können und bei möglichst geringem Herstellaufwand und einfacher Anwendung dem Anwender bzw. Patienten eine Rückmeldung zu geben, dass die gewünschte Wirkung der Wundabdeckung aktiviert ist, ist der Einsatz eines bi-stabil verformbaren Druckelements vorgesehen.

## Beschreibung

Die Erfindung betrifft eine Wundabdeckung, insbesondere eine Wundabdeckung in der Form eines auf die Haut aufzuklebenden Pflasters (Wundschnellverband), mit einem Träger bzw. einer Trägerlage, der bzw. die eine auf die Haut eines Nutzers anzuordnende und dieser bei bestimmungsgemäßer Anwendung zugewandten Unterseite und eine von der Haut weg weisende Oberseite aufweist, wobei an der Unterseite des Trägers bzw. der Trägerlage ein bevorzugt steriles Abdeckpolster zum Auflegen auf einen Wundbereich der Haut angeordnet ist. Das Abdeckpolster bildet die bevorzugt unmittelbar mit der Haut bzw. der Wunde in Kontakt kommende Kontaktlage der Wundabdeckung. Ein Druckelement kann insbesondere oberhalb einer dem Wundbereich zugewandten Unterseite des Abdeckpolsters bzw. der Kontaktlage vorgesehen sein. Unter dem Begriff "Wundbereich" ist insbesondere auch eine lediglich punktuell vorliegende kleinere Wunde wie ein kleiner Schnitt oder eine Einstichstelle zu verstehen,

Eine als Druckpflaster ausgebildete Wundabdeckung dieser Art ist aus der DE 94 03 721 U1 bekannt. Bei dieser Wundabdeckung handelt es sich im Wesentlichen um einen klassischen Druckverband, der ein Druckelement auf die Haut oberhalb einer blutenden Verletzung drückt.

Solche Druckverbände sind im Falle von starken Blutungen hilfreich, jedoch zum einen bei kleineren Wunden bzw. Einstichstellen, die infolge einer medizinischen Behandlung oder einer Blutspende entstanden sind, nicht zwingend notwendig und außerdem unangenehm zu tragen. Ferner weisen sie den Nachteil auf, dass der Benutzer bzw. die Pflegekraft keine hinreichende Rückmeldung dahingehend erhält, ob das unter dem Pflastermaterial befindliche Druckelement tatsächlich die Blutung stillt oder ob das Blut infolge eines zu geringen Druckes an dem Druckelement in den umgebenden Bereich sickert.

Aus der CN 202 458 906 ist ein Druckpflaster bekannt, bei dem das Abdeckpolster über eine Feder, die zwischen dem Abdeckpolster und einer äußeren Trägerschicht angeordnet ist, gegen die Wunde gedrückt wird. Diese Lösung ist zum einen aufwendig herzustellen und weist darüber hinaus einen geringen Tragekomfort auf, da die Feder vergleichsweise viel Platz in Anspruch nimmt und nicht derart elastisch ist, so dass sie sich an Krümmungen der Haut anpassen kann. Für kleinere Wundbereiche, wie zum Beispiel kleinere Schnitte oder Einstichwunden, wie sie bei der Blutabnahme oder Blutspende oder bei der Dialyse entstehen, ist diese Lösung somit kaum geeignet.

Als Druckpflaster konzipierte Wundabdeckungen sind auch in den Druckschriften US 4,377,159, WO 2004/112666 A1 und EP 0 614 652 B1 offenbart.

Aufgabe der Erfindung ist es, eine Wundabdeckung zu schaffen, die bei möglichst geringem Herstellaufwand und einfacher Anwendung insbesondere kleinere Blutungen (arteriell oder venös) durch entsprechenden Druck des Abdeckpolsters auf den Wundbereich stillen kann und dem Anwender bzw. Patienten möglichst eine bevorzugt hörbare und/oder fühlbare Rückmeldung gibt, dass die Wirkung der Wundabdeckung aktiviert ist.

Diese Aufgabe wird nach der Erfindung dadurch gelöst, dass im Bereich der Kontaktlage, insbesondere also im Bereich des Abdeckpolsters und oberhalb einer dem Wundbereich zugewandten Unterseite des Abdeckpolsters, ein hinreichend elastisch bi-stabil verformbares Druckelement angeordnet ist.

Dieses Druckelement ist zumindest in einer Richtung relativ zur Ebene des Trägers bzw. der Trägerlage derart gebogen, dass es zur Bildung einer von oben gesehen konvexen, nicht druckausübenden Form mit wenigstens einem mittleren Druckbereich nach oben ragt. In der nicht Druck ausübenden (inaktiven) Form bildet das Druckelement eine von der Haut weg gerichtete Erhebung. Das Druckelement ist derart bi-stabil ausgebildet, dass es unter Verformung in Richtung der Unterseite des Trägers von der nicht druckausübenden, konvexen Form in eine druckausübende, konkave Form überführbar ist, in der es das Abdeckpolster zumindest abschnittweise nach unten in Richtung auf die Haut des Nutzers drückt. In der Druck ausübenden (aktiven) Form bildet das Druckelement demnach eine zur Haut hin gerichtete Erhebung, mittels der die über den auf der Haut des Patienten befindlichen Trägers ein gegenüber einem Wundverband ohne ein derartiges Druckelement gesteigerter Druck auf den Wundbereich ausgeübt werden kann.

Erfindungsgemäß ist nun mit dem bi-stabilen Druckelement ein einfach herstellbares Bauteil als Teil der Wundabdeckung vorgesehen, das leicht und einfach von einer inaktiven Stellung in eine aktive Stellung überführt werden kann. Hierzu ist das Druckelement in der inaktiven Stellung, in der es keinen Druck auf die Haut des Nutzers ausübt, zumindest in einer Richtung konvex ausgebildet (das Druckelement ist nach oben, von der Haut des Nutzers weg gewölbt). In dieser inaktiven Stellung ist von oben gesehen ein mittlerer Bereich des Druckelementes erhaben. Dies kann eine punktförmige Erhebung sein, so dass das Druckelement in einem solchen Fall in etwa die Form einer Glocke oder eines Segmentes einer hohlen Kugel oder eines Ellipsoiden aufweist, also kalottenförmig ausgebildet ist. Alternativ kann auch eine linienförmige Erhebung, die sich über die gesamte Breite des Druckelementes erstrecken kann, vorgesehen sein. Bevorzugt ist das Druckelement aber als kalottenförmiges, bi-stabil verformbares und bevorzugt rundes Bauteil nach Art eines kalottenartig gewölbten Plättchens ausgebildet.

Das Druckelement ist bi-stabil ausgebildet, so dass es unter Deformation von der konvexen Form (inaktive Stellung) in eine konkave Form (aktive Stellung) überführt werden kann, dabei die konkave Form bzw. aktive Stellung jedoch selbsttätig und dauerhaft beizubehalten vermag. Dies bewirkt, dass der erhabene, mittlere Bereich in der aktiven Stellung nicht weiter von der Haut weg gerichtet ist, sondern gegen die Haut zu drücken vermag. Hierzu ist das Druckelementes mit dem Träger bzw. der Trägerlage der Wundabdeckung derart verbunden, dass die Druckkraft über den Träger bzw. die Trägerlage auf das Druckelement und von diesem bevorzugt über die Kontaktlage (das Abdeckpolster) auf die Haut des Nutzers weitergeleitet ist.

Im Falle eines Druckelementes, das die genannte punktförmige Erhebung aufweist, sind hierzu insbesondere die Randbereiche des Druckelements mit dem Träger oder alternativ bzw. zusätzlich mit der Kontaktlage bzw. dem Abdeckpolster verbunden, etwa verklebt oder verschweißt. Ist dagegen das Druckelement mit einer sich linienförmig verlaufenden Erhebung versehen, kann es ausreichend sein, wenn die Ränder zu beiden Seiten der linienförmigen Erhebung in Querrichtung, also parallel zur Hautoberfläche gehalten sind.

In allen Fällen ist das Druckelement so ausgebildet, dass es durch Eindrücken von einer ersten stabilen, inaktiven Stellung, in der der erhabene Bereich des Druckelementes von der Haut weg gerichtet ist, in eine zweite stabile, aktive Stellung überführt werden kann, in der der erhabene Bereich des Druckelements der Haut zugewandt ist. Hierzu wird das Druckelement kurzfristig über eine Nulllage hinweg elastisch verformt, bis es in die entgegengesetzte Richtung wieder ausfedert. Bei geeigneter Materialwahl kann dies nicht nur fühlbar sein, sondern auch ein akustisches Klicken erzeugen, das vom Benutzer oder von der Pflegekraft als Rückmeldung, dass das Druckelement erfolgreich aktiviert wurde, wahrnehmbar ist. So kann eine optische, fühlbare und hörbare Rückmeldung über die Aktivierung der Funktion des Druckelementes auf einfache Art und Weise realisiert werden.

Bei einer bevorzugten Ausgestaltung ist die Wandstärke des Druckelementes über seine Quer- bzw. Längsausdehnung im Wesentlichen gleichbleibend. Insbesondere im Falle eines kalottenförmigen oder glockenartigen Druckelementes kann so einfach die bi-stabile Verformbarkeit realisiert werden, wenn der Werkstoff des Druckelementes für die beabsichtigte Verformung ausgehend von der inaktiven Stellung hinreichend biegeelastisch ist. Andererseits muss der Werkstoff des Druckelementes auch derart fest sein, dass das Druckelement in der Lage ist, den notwendigen Druck auf den Wundbereich auszuüben, um die Blutung zu stoppen. Die Materialstärke "s" des Druckelements ist bevorzugt kleiner oder gleich 1 mm, weiter bevorzugt kleiner oder gleich 0,5 mm. Als besonders hat sich eine Materialstärke "s" zwischen 0,3 mm und 0,5 mm, insbesondere um 0,4 mm erwiesen.

Ein bevorzugter Werkstoff für die Herstellung des Druckelementes ist Kunststoff. Dieser ist zum einen hinreichend verformbar, um die notwendige Deformation zerstörungsfrei auszuhalten, zum anderen kann Kunststoff in den beiden stabilen Zuständen auch die notwendigen Kräfte aufnehmen bzw. auf die Haut übertragen. Es kommen aber auch anderen Materialien in Betracht, sofern diese die geforderte Verformbarkeit ausgehend von der Form, die das Druckelement in der inaktiven Stellung einnimmt, gewährleisten können. Der Träger ist bevorzugt pflasterartig als textile oder textilartige Stoff-, Kunstfaser- oder Kunststofffolienlage ausgebildet, so dass er auf die Haut aufgeklebt werden kann. Im Bereich des Druckelementes ist natürlich keine Klebeschicht vorgesehen, vielmehr befindet sich unter dem Druckelement das Abdeckpolster, das auf den Wundbereich aufgelegt wird.

Das Druckelement und das Abdeckpolster bzw. die Kontaktlage und ggf. sogar der Träger können von einem gemeinsamen Bauteil oder einem zusammenhängenden Bauteilverbund gebildet sein. Hierzu müsste zum Beispiel eine saugfähige Schicht auf ein Trägermaterial kaschiert werden, so dass das Trägermaterial die notwendigen Eigenschaften zur Druckausübung des Druckelementes beisteuert, während die aufkaschierte, saugfähige Schicht die Funktionen des Abdeckpolsters übernimmt. Bei entsprechender Materialwahl kann auch ein einzelnes Bauteil die Funktion des Druckelementes und des Abdeckpolsters übernehmen, ohne dass dieses aus mehreren Materialien bestehen muss. Hier käme beispielsweise ein elastischer, über einen bestimmten Bereich aber dennoch formstabiler bzw. hinreichend bi-stabiler Schaum in Betracht. Üblicherweise wird zur Erzielung einer größeren Abdeckfläche des Wundbereiches jedoch ein separates Abdeckpolster bzw. eine separate Kontaktlage bevorzugt sein.

Anstelle einer pflasterartigen Wundabdeckung kann natürlich auch jede andere Form von Verband als Träger gewählt werden. Das zunächst konvexe und im aktiven Zustand konkave (jeweils von oben auf die Haut gesehen) Druckelement kann auch in einem klassischen Verband, der zum Beispiel um einen Arm oder ein sonstiges Körperteil einer Person gewickelt wird, enthalten bzw. in diesem in geeigneter Form eingebunden sein.

Das Druckelement kann fest mit dem Träger und/oder dem Abdeckpolster verbunden sein. Insbesondere kann vorgesehen sein, dass das Druckelement mit einem Teil der Wundabdeckung, insbesondere mit dem Träger und/oder der Kontaktlage, stoffschlüssig verbunden, insbesondere verklebt oder verschweißt ist. Alternativ kann der Träger auch eine Tasche aufweisen, in die das Druckelement bei Bedarf insbesondere seitlich eingesteckt werden kann. In einem solchen Fall kann die Tasche geschlossen sein, so dass das Druckelement nicht entnommen werden kann. Alternativ kann die Tasche auch insbesondere einseitig offen sein, so dass zum Beispiel das Druckelement nur bei Bedarf eingeschoben wird, ansonsten das Pflaster oder der Verband aber wie ein herkömmliches Pflaster bzw. herkömmlicher Verband benutzt werden kann. Im Falle eines Verbandes kann das Druckelement auch einfach durch Einwickeln in den Verband integriert werden. In diesem Fall empfiehlt sich die Verwendung eines punktförmig erhabenen Druckelementes, das dann einfach durch Druck auf den mittleren Bereich in die konkave, aktive Position überführt werden kann.

Rund um das Druckelement bzw. in dem Bereich in dem seine Ränder bei bestimmungsgemäßer Verwendung platziert werden, kann der Träger einen Verstärkungsring oder Verstärkungsrahmen aufweisen, über den die von den Randbereichen des Druckelementes auf das Trägermaterial aufgebrachten Kräfte in den Träger eingeleitet werden können. Ein solcher Verstärkungsring oder Verstärkungsrahmen hat den Vorteil, dass die Kräfte des Druckelementes das üblicherweise leichte Material des Trägers nicht übermäßig belasten.

Bei einer Ausführungsform des Druckelements kann vorgesehen sein, dass das Druckelement einen Randbereich aufweist, der insbesondere auch flach bzw. eben (der Wölbung eines inneren Abschnitts des Druckelements nicht folgend) ausgebildet sein kann. Ein derartiger Randabschnitt kann einfacher von in einer automatisierten Fertigung typischerweise verwendeten Werkzeugen gegriffen und auch besser mit den übrigen Teilen der Wundabdeckung verbunden werden, insbesondere verklebt oder verschweißt werden. Ein derartiger Randabschnitt kann insbesondere bei einem kalottenförmig ausgebildeten Druckelement umlaufend sein. Die bevorzugte Breite "b" eines derartigen Randbereichs ist kleiner oder gleich 2 mm, bevorzugt zwischen 0,5 mm und 1,5 mm, insbesondere etwa 1 mm.

Weitere Merkmale und Vorteile der Erfindung ergeben sich der nachfolgenden Beschreibung bevorzugter Ausführungsbeispiele anhand der Zeichnungen.

In den Zeichnungen zeigt:
- Fig. 1: eine dreidimensionale, schematische Ansicht einer erfindungsgemäßen Wundabdeckung,
- Fig. 2: eine weitere erfindungsgemäße Wundabdeckung,
- Fig. 3: eine Seitenansicht einer Wundabdeckung, bei der sich das Druckelement in einer nicht druckausübenden, inaktiven Stellung befindet,
- Fig. 4: die in Figur 3 dargestellte Wundabdeckung, bei der sich das Druckelement in einer druckausübenden, aktiven Stellung befindet, und
- Fig. 5: ein von einer Wundabdeckung losgelöstes Druckelement.

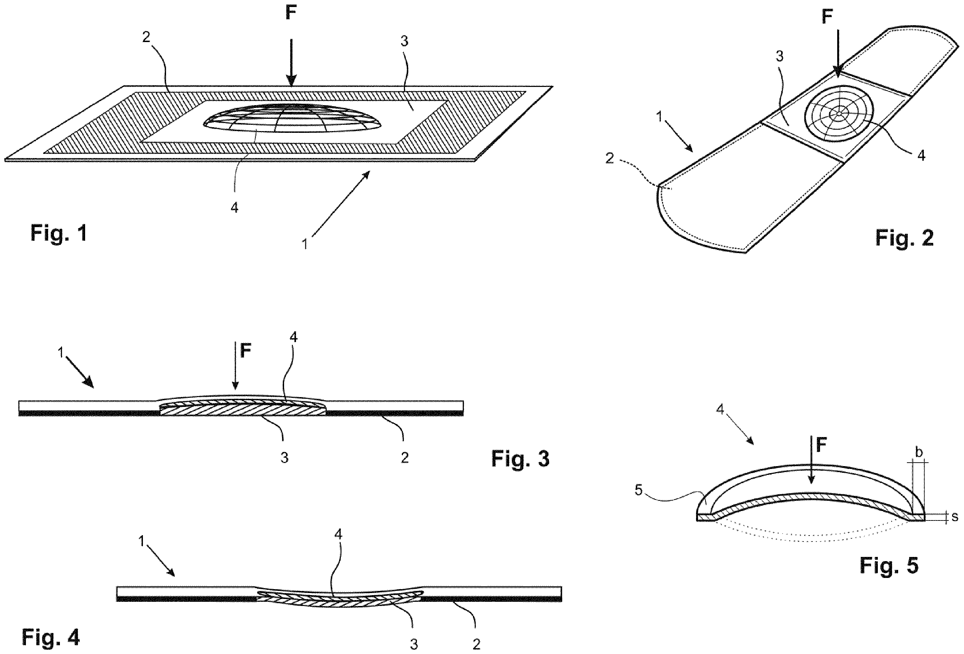

In Figur 1 ist eine erfindungsgemäße Wundabdeckung schematisch dargestellt. Die Wundabdeckung besteht aus einem Träger 1, der an seiner Unterseite eine adhäsive Schicht 2 aufweist. Diese Wundabdeckung ist nach der Art eines herkömmlichen Wundschnellverbands (Pflaster) ausgebildet.

Im mittleren Bereich weist die Wundabdeckung an ihrer Unterseite als Kontaktlage ein Abdeckpolster 3 auf. Dieses Abdeckpolster 3 ist beispielsweise eine übliche, saugfähige Gaze-Schicht, die auf den Wundbereich aufgelegt wird, ggf. mit einer antibakteriell wirksamen Beschichtung. Über die adhäsive Schicht 2 wird der Träger 1 dann rund um den Wundbereich auf die Haut des Trägers geklebt.

Oberhalb des Abdeckpolsters 3 ist, hier beispielhaft als linsen- oder kugelsegmentartiges rundes Plättchen ausgebildet, das Druckelement 4 vorgesehen. Dieses Druckelement 4 weist einen mittleren, erhabenen Bereich auf und ist hier in der inaktiven Stellung dargestellt, in der es keinen Druck auf den unterhalb des Druckelementes 4 liegenden Bereich des Abdeckpolsters 3 ausübt. Dies entspricht dem Auslieferungszustand der Wundabdeckung.

Figur 2 zeigt in einer anderen, dreidimensionalen Darstellung eine ähnliche Wundabdeckung, bei der sich das Abdeckpolster 3 über die gesamte Breite des Trägers 1 erstreckt. Zu beiden Seiten des Abdeckpolsters 3 erstreckt sich der Träger 1 mit Flügeln, an deren Unterseite die adhäsive Schicht 2 vorgesehen ist. Auch hier ist das Druckelement 4 punktförmig erhaben und in der inaktiven Stellung gezeigt.

Die Figuren 3 und 4 verdeutlichen die Funktionsweise der in den Figuren 1 und 2 dargestellten Wundabdeckungen:
Figur 3 zeigt den inaktiven Zustand. In diesem Zustand ist das oberhalb des Abdeckpolsters 3 angeordnete Druckelement 4 von oben gesehen konvex geformt und bildet eine von der Haut weg gerichtete erhabene Wölbung aus. Der Träger 1 deckt sowohl die adhäsive Schicht 2 als auch das Druckelement 4 ab und ist aus einem üblichen Pflastermaterial gefertigt. Zur Führung des Druckelementes 4 in die aktive Stellung wird nun eine Betätigungskraft F in Pfeilrichtung auf das Druckelement 4 aufgebracht. Dies kann vor dem Aufkleben der Wundabdeckung auf die Haut oder - aus den nachfolgend genannten Gründen bevorzugt - danach erfolgen.

In Figur 4 ist der aktive Zustand des Druckelementes 4 dargestellt. Zu erkennen ist, dass die ehemals konvexe Form des Druckelementes 4 nun in eine konkave Form umgewandelt wurde, die das Druckelement aufgrund seiner Bi-Stabilität aber ebenso selbsttätig und dauerhaft beizubehalten vermag wie die inaktive, konvexe Form. Das Druckelement 4 weist über seinen gesamten Bereich in etwa die gleiche Dicke auf und kann durch Eindrücken von der in Figur 3 gezeigten ersten stabilen Stellung in die weitere, in Figur 4 gezeigte zweite stabile Stellung überführt werden (Bi-Stabilität). Die Wundabdeckung drückt jetzt mit einer der Haut zugewandten erhabenen Wölbung des Druckelementes 4 das Abdeckpolsters 3 gegen die Haut rund um den Wundbereich.

Beim Überspringen von der in Figur 3 gezeigten inaktiven Form in die in Figur 4 gezeigte aktive Form wird das Druckelement bei geeigneter Materialauswahl sowohl ein fühl- als auch hörbares Feedback an den Benutzer geben, sobald es durch die Nulllage hindurch in die aktive Stellung schnellt.

Damit hier die entsprechenden Druckkräfte aufgebracht werden können, ist es empfehlenswert, die Wundabdeckung vor dem Eindrücken des Druckelementes 4 auf die Haut zu kleben, so dass die adhäsive Schicht 2 bereits fest mit der Haut verbunden ist und die sich infolge des Umschlagens des Druckelementes 4 von der konvexen inaktiven in die konkave aktive Form erhöhende auf die Haut wirkende Druckkraft vollständig auf den Wundbereich wirkt. Zudem wirkt das Eindrücken des Druckelements selbst durch die Wundabdeckung hindurch zumindest kurzzeitig in vorteilhafter Weise auf zum Beispiel die Einstichstelle ein.

Figur 5 zeigt ein von der Wundabdeckung losgelöstes inaktives Druckelement 4 in einer perspektivischen Ansicht im Schnitt. Die aktive Position ist zur Veranschaulichung gestrichelt dargestellt. Das Druckelement 4 weist etwa eine Materialstärke "s" von 0,4 mm auf. Ein mittlerer erhabener Wölbungsbereich ist von einem umlaufenden ebenen Randbereich 5 umgeben, der bevorzugt etwa eine Breite "b" von 0,3 mm bis 0,5 mm aufweist und zur Verbindung mit einem Teil und/oder einer Lage der Wundabdeckung dient. Insbesondere kann das Druckelement durch das Vorsehen eines solchen Randbereichs gut mit einem Teil und/oder einer Materiallage der Wundabdeckung verklebt oder (ultraschall)verschweißt werden. Ein derartiger Randbereich, der nicht notwendigerweise umlaufend sein muss, vereinfacht insbesondere die automatisierte Herstellung einer Wundabdeckung und das Einwirken von Werkzeugen auf das Druckelement, etwa von Ultraschallköpfen.

### Bezugszeichenliste

- 1: Träger
- 2: Adhäsive Schicht
- 3: Kontaktlage/Abdeckpolster
- 4: Druckelement
- 5: Randbereich

- F: Druckkraft

## Patentansprüche

1. Wundabdeckung mit einer Kontaktlage (3) zum Auflegen auf einen Wundbereich der Haut, **dadurch gekennzeichnet, dass**
im Bereich der Kontaktlage (3) ein bi-stabil verformbares Druckelement (4) vorgesehen ist.

2. Wundabdeckung nach Anspruch 1, **gekennzeichnet durch** einen Träger (1), der eine der Haut zuzuwendende Unterseite und eine Oberseite aufweist, wobei die Kontaktlage (3) an der Unterseite des Trägers (1) angeordnet oder der Unterseite zwecks Schaffung einer derartigen Kontaktlage (3) der Wundabdeckung zuzuordnen ist.

3. Wundabdeckung nach Anspruch 1 oder 2, **dadurch gekennzeichnet, dass** das Druckelement relativ zu zumindest einer, zur Oberfläche eines Trägers (1) parallelen Richtung derart gebogen ist, dass es zur Bildung einer von oben gesehen konvexen, nicht druckausübenden Form mit wenigstens einem mittleren Druckbereich nach oben ragt, und derart ausgebildet ist, dass es unter Verformung in Richtung der Unterseite des Trägers von der nicht druckausübenden, konvexen Form in eine druckausübende, konkave Form überführbar ist, in der es die Kontaktlage (3) zumindest abschnittweise nach unten drückt.

4. Wundabdeckung nach einem der vorhergehenden Ansprüche, **dadurch gekennzeichnet, dass** das Druckelement (4) jeweils von unten gesehen zumindest in einer Richtung in der nicht druckausübenden Form eine konkave Form und in der druckausübenden Form eine konvexe Form aufweist.

5. Wundabdeckung nach einem der vorhergehenden Ansprüche, **dadurch gekennzeichnet, dass** das Druckelement (4) einen erhabenen mittleren Bereich aufweist, vom dem aus es sich in alle Richtungen zu einem relativ zum mittleren Bereich tieferliegenden Randbereich erstreckt.

6. Wundabdeckung nach einem der Ansprüche 1 bis 4, **dadurch gekennzeichnet, dass** das Druckelement (4) sich in einer Längsrichtung von einem erhabenen mittleren Bereich zu zwei sich gegenüberliegenden, relativ zum mittleren Bereich tiefer liegenden Randbereichen erstreckt und die Randbereiche zumindest in Längsrichtung an dem Träger (1) oder der Kontaktlage (3) derart gehalten sind, dass sie nicht in eine von dem mittleren Bereich wegweisende Richtung auswandern können.

7. Wundabdeckung nach einem der vorhergehenden Ansprüche, **dadurch gekennzeichnet, dass** die Wundabdeckung eine Tasche aufweist, in die das Druckelement (4) eingelegt oder einlegbar ist.

8. Wundabdeckung nach dem vorhergehenden Anspruch, **dadurch gekennzeichnet, dass** die Tasche eine insbesondere verschließbare Öffnung aufweist, die derart ausgebildet ist, dass das Druckelement (4) aus der Tasche entnehmbar oder in diese einführbar ist.

9. Wundabdeckung nach einem der Ansprüche 1 bis 7, **dadurch gekennzeichnet, dass** das Druckelement (4) fest mit der Kontaktlage (3) verbunden ist, insbesondere mit dieser verlebt oder verschweißt ist.

10. Wundabdeckung nach einem der Ansprüche 1 bis 7 oder nach Anspruch 9, **dadurch gekennzeichnet, dass** das Druckelement (4) fest mit dem Träger (1) verbunden ist, insbesondere mit diesem verklebt oder verschweißt ist.

11. Wundabdeckung nach einem der vorhergehenden Ansprüche, **dadurch gekennzeichnet, dass** der Träger (1) als Pflaster ausgebildet ist, das an seiner Unterseite seitlich neben einer Kontaktlage (3) eine adhäsive Schicht aufweist.

12. Wundabdeckung nach dem vorhergehenden Anspruch, **dadurch gekennzeichnet, dass** der Träger (1) zumindest im Bereich des Druckelementes (3) elastisch ausgebildet ist, wobei bei druckausübender Form des Druckelementes (3) die Klebekraft der adhäsiven Schicht (2) größer als die elastischen Rückstellkräfte des Trägers (1) ist.

13. Wundabdeckung nach einem der Ansprüche 1 bis 7 oder 10 bis 12, **dadurch gekennzeichnet, dass** das Druckelement (4) auf dem Träger (1) angeordnet ist.

14. Wundabdeckung nach einem der vorhergehenden Ansprüche, **dadurch gekennzeichnet, dass** das Druckelement (4) von einem bi-stabil verformbaren Plättchen gebildet ist.

15. Verwendung eines bi-stabil verformbaren Druckelements (4), insbesondere eines Druckelements nach einem der vorhergehenden Ansprüche, für eine Wundabdeckung zwecks Aufbringung einer gesteigerten Druckkraft auf einen Wundbereich in der Haut eines Nutzers, wobei das Druckelement (4) durch Verformung von einer ersten stabilen Form, in der es keine zur Haut hin gerichtete Erhebung bildet, durch Verformung in eine zweite stabile Form überführbar ist, in der es eine zur Haut hin gerichtete Erhebung bildet.
